# EUROPEAN PATENT APPLICATION

(11) **EP 4 818 335 A1**
(43) Date of publication of application: **07.10.2026**
(21) Application number: 24897850.4
(22) Date of filing: 30.09.2024
(51) Int. Cl.: C12Q 1/689

(54) **COMPOSITION FOR DETECTING ROTHIA KRISTINAE YM-6 STRAIN, AND USE THEREOF**

(30) Priority: 01.12.2023 KR 20230172606
(71) Applicant: Cosmax, Inc., Hwaseong-si, Gyeonggi-do 18622 (KR)
(72) Inventor: KIM, Hye Been, Seongnam-si, Gyeonggi-do 13486 (KR); LEE, Ha Eun, Seongnam-si, Gyeonggi-do 13486 (KR); LEE, Dong Geol, Seongnam-si, Gyeonggi-do 13486 (KR); KANG, Seung Hyun, Seoul 06285 (KR)
(74) Representative: Croce, Valeria
(86) International application number: PCT/KR2024/014864
(87) International publication number: WO 2025/116251

(57) **Abstract**

Provided are a composition for detecting a *Rothia kristinae* YM-6 strain, a primer set for detecting a *Rothia kristinae* YM-6 strain, and a detection method using the composition and the primer set. The composition can rapidly and accurately detect or identify a *Rothia kristinae* YM-6 strain with high detection specificity at strain or species level.

## Description

### Technical Field

The present disclosure relates to a composition for identifying a microorganism at a specific strain level and a use thereof.

### Background Art

The skin is the largest organ of the human body in contact with the external environment. The skin protects the body from external stimuli, and has a core function of preventing moisture from evaporating from the skin. Along with other body organs such as intestines, various microorganisms such as bacteria, fungi, and viruses, as well as small organisms such as small larvae, inhabit the skin while maintaining specialized functions. Such microorganisms present on the skin and the entire genetic information thereof are defined as the skin microbiome.

Skin microbiome research has become possible with the development of next-generation sequencing (NGS) analysis methods. In particular, when a 16S rRNA gene primer is utilized, the bacterial composition of microflora can be cataloged and processed.

Such a 16S rRNA gene-based NGS analysis method has an advantage of being capable of detecting all microorganisms in a specific environment, but has limitations in that it takes at least one week to date and a specific strain cannot be detected with the 16S rRNA gene. In addition, there is a disadvantage in that detecting a specific strain utilizing NGS requires high costs.

(Patent Document 1) KR 10-2286080 B1

### Disclosure of Invention

### Technical Problem

The present specification relates to a whole genome sequence (WGS) data-based molecular marker capable of identifying the *Rothia kristinae* YM-6 strain at strain or species level. By using the molecular marker, the *Rothia kristinae* YM-6 strain can be identified relatively rapidly and accurately through a nucleic acid amplification reaction such as a polymerase chain reaction (PCR).

One aspect is to provide a composition for detecting the *Rothia kristinae* YM-6 strain deposited under accession number KCCM12685P, including an agent capable of specifically detecting a polynucleotide consisting of SEQ ID NO: 1 or a complementary polynucleotide thereof.

Another aspect is to provide a primer set for detecting the *Rothia kristinae* YM-6 strain deposited under accession number KCCM12685P, consisting of a forward primer set forth in SEQ ID NO: 2 and a reverse primer set forth in SEQ ID NO: 3.

Another aspect is to provide a kit for detecting the *Rothia kristinae* YM-6 strain deposited under accession number KCCM12685P, including the composition according to one aspect.

Another aspect is to provide a method of detecting the *Rothia kristinae* YM-6 strain deposited under accession number KCCM12685P using the composition, the primer set, or the kit according to one aspect.

### Solution to Problem

One aspect provides a composition for detecting the *Rothia kristinae* YM-6 strain deposited under accession number KCCM12685P, including an agent capable of specifically detecting a polynucleotide consisting of SEQ ID NO: 1 or a complementary polynucleotide thereof.

The *Rothia kristinae* YM-6 strain deposited under accession number KCCM12685P is disclosed in Korean Patent Registration No. 10-2286080. The strain deposited under accession number KCCM12685P may be a *Rothia kristinae* subsp. *acnes* YM-6 strain.

The polynucleotide consisting of SEQ ID NO: 1 or the complementary polynucleotide thereof may be a partial sequence of the whole genome sequence of the *Rothia kristinae* YM-6 strain deposited under accession number KCCM12685P. The whole genome includes all genetic information of the corresponding microorganism, such as chromosomal DNA and plasmid DNA. SEQ ID NO: 1 may be the sequence at positions 5311 to 5623 of the complete sequence of the chromosomal DNA of the YM-6 strain.

The polynucleotide consisting of SEQ ID NO: 1 or the complementary polynucleotide thereof may be a whole genome sequence (WGS)-based molecular marker capable of identifying the *Rothia kristinae* YM-6 strain at strain or species level. The "molecular marker" refers to a molecule having a characteristic capable of confirming the presence or state of a certain organism or substance. For example, a specific DNA sequence may be used as a molecular marker capable of determining the presence or absence of a specific strain. When the sequence of SEQ ID NO: 1 or a complementary sequence thereof is present in a nucleic acid sequence of a microorganism of interest, the microorganism may be identified as the *Rothia kristinae* YM-6 strain.

In addition, since the polynucleotide consisting of SEQ ID NO: 1 or the complementary polynucleotide thereof is a whole genome sequence (WGS)-based molecular marker, detection specificity is significantly superior compared to a conventional 16S rRNA gene-based NGS analysis method incapable of detecting a specific strain. Therefore, by utilizing the molecular marker, the *Rothia kristinae* YM-6 strain can be distinguished from microorganisms belonging to closely related strains or species that are otherwise indistinguishable by general physiological or chemical differences.

Furthermore, when an agent capable of specifically detecting the polynucleotide consisting of SEQ ID NO: 1 or the complementary polynucleotide thereof is used, a specific strain can be identified merely by detecting a molecular marker having a relatively short length (e.g., 313 bp), and thus time and costs can be significantly reduced compared to a conventional NGS analysis method that examines a 16S rRNA gene having a length of hundreds to thousands of bp.

Accordingly, the agent capable of specifically detecting the polynucleotide consisting of SEQ ID NO: 1 or the complementary polynucleotide thereof may be an agent capable of detecting or identifying the *Rothia kristinae* YM-6 strain. The agent can specifically detect only the *Rothia kristinae* YM-6 strain by distinguishing the strain even from microorganisms with finely divergent phylogenetic lineages from the *Rothia kristinae* YM-6 strain. Therefore, the composition according to one aspect may be a composition for identifying the *Rothia kristinae* YM-6 strain deposited under accession number KCCM12685P.

The type of the agent is not limited as long as the agent is a substance capable of specifically binding to the polynucleotide or the complementary polynucleotide thereof. The agent may be a primer or a probe specifically binding to the polynucleotide or the complementary polynucleotide thereof, but is not limited thereto.

The "primer" is a short single-stranded oligonucleotide serving as a starting point from which another polymer strand complementary to a template is generated during DNA synthesis. A primer used in a nucleic acid amplification reaction may include a primer set consisting of a forward primer and a reverse primer. The length of the primer may be 15 to 40 nt, 15 to 30 nt, 15 to 25 nt, 18 to 40 nt, 18 to 30 nt, or 18 to 25 nt, but is not limited thereto.

When a nucleic acid amplification reaction is performed using a primer specifically binding to the polynucleotide consisting of SEQ ID NO: 1 or the complementary polynucleotide thereof, the *Rothia kristinae* YM-6 strain can be specifically identified according to the presence or absence of an amplification product.

In an embodiment, the primer may be a primer set consisting of a forward primer set forth in SEQ ID NO: 2 and a reverse primer set forth in SEQ ID NO: 3.

The "probe" refers to a DNA or RNA fragment complementary to a specific base sequence of DNA or RNA, and the presence or absence of the specific base sequence can be confirmed since the probe is labeled with a radioactive element, a dye, a fluorophore, or the like. The length of the probe may be 10 to 1000 bp, 10 to 500 bp, 10 to 300 bp, 10 to 200 bp, 10 to 100 bp, 50 to 1000 bp, 50 to 500 bp, 50 to 300 bp, 50 to 200 bp, 50 to 100 bp, 100 to 1000 bp, 100 to 500 bp, 100 to 300 bp, 100 to 200 bp, 150 to 1000 bp, 150 to 500 bp, 150 to 300 bp, 150 to 200 bp, 200 to 1000 bp, 200 to 500 bp, 200 to 300 bp, 250 to 1000 bp, 250 to 500 bp, 250 to 350 bp, or 250 to 300 bp, but is not limited thereto.

When hybridization is performed using a probe specifically binding to the polynucleotide consisting of SEQ ID NO: 1 or the complementary polynucleotide thereof, the *Rothia kristinae* YM-6 strain can be specifically identified based on whether or not hybridization occurs.

In an embodiment, the probe may be an oligonucleotide consisting of a contiguous nucleotide sequence of 10 or more, 20 or more, 30 or more, 40 or more, 50 or more, 100 or more, 150 or more, 200 or more, 250 or more, or 300 or more nucleotides complementary to part or all of "the polynucleotide consisting of SEQ ID NO: 1 or the complementary polynucleotide thereof".

In an embodiment, the probe may be an oligonucleotide consisting of a nucleotide sequence having a length of 313 nt complementary to "the polynucleotide consisting of SEQ ID NO: 1 or the complementary polynucleotide thereof".

The primer or the probe can be chemically synthesized using a known method. The primer or the probe may further include at least one label linked to the 5'-end or the 3'-end and selected from a radioactive element, a chromophore, a chemiluminophore, a fluorophore, and a magnetic particle.

The "label" or "detectable label" may refer to any chemical moiety bound to a nucleotide or a nucleotide polymer, and the bond may be a covalent bond or a noncovalent bond. The detectable label is known in the art. Non-limiting examples of the detectable label include radioactive elements such as ³²P or ³⁵S; linker molecules such as biotin, avidin, streptavidin, horseradish peroxidase (HRP), protein A, protein G, an antibody or a fragment thereof, a FLAG tag, and a myc tag; enzymes such as peroxidase and luciferase; electron donors and acceptors; dyes such as Cy5 and Cy3; and magnetic particles (MPs) such as iron oxide nanoparticles and gold nanoparticles.

Another aspect provides a primer set for detecting the *Rothia kristinae* YM-6 strain deposited under accession number KCCM12685P, consisting of a forward primer set forth in SEQ ID NO: 2 and a reverse primer set forth in SEQ ID NO: 3.

The forward primer set forth in SEQ ID NO: 2 has a length of 20 bp, a melting temperature (Tm) of about 60 °C, and a GC % of about 50 %.

The reverse primer set forth in SEQ ID NO: 3 has a length of 20 bp, a Tm of about 60 °C, and a GC % of about 55 %.

When a nucleic acid amplification reaction is performed using the primer set, an amplification product having a size of 313 bp can be detected only when the *Rothia kristinae* YM-6 strain is present.

In one example, when PCR was performed using the primer set of SEQ ID NO: 2 and SEQ ID NO: 3, it was confirmed that under a single condition having an identical annealing temperature and time, no PCR product was synthesized for the *Kocuria varians* YM-3 and *Kocuria rhizophila* KACC 14744 strains, which belong to the same *Micrococcaceae* family but differ in both genus and species, and a PCR product was synthesized exclusively for the *Rothia kristinae* YM-6 strain. These results confirm that even when a strain of a different genus is present in a sample, only the *Rothia kristinae* YM-6 strain can be specifically and clearly detected and identified at genus level.

In one example, when PCR was performed using the primer set of SEQ ID NO: 2 and SEQ ID NO: 3, it was confirmed that under a single condition having an identical annealing temperature and time, no PCR product was synthesized for the *Rothia koreensis* KACC 17049 strain belonging to the same genus, and a PCR product was synthesized exclusively for the *Rothia kristinae* YM-6 strain. These results confirm that even when a strain of the same genus is present in a sample, only the *Rothia kristinae* YM-6 strain can be specifically and clearly detected and identified at species level.

In one example, when PCR was performed using the primer set of SEQ ID NO: 2 and SEQ ID NO: 3, it was confirmed that under a single condition having an identical annealing temperature and time, no PCR product was synthesized for the *Rothia kristinae* KACC 15198 strain belonging to the same species, and a PCR product was synthesized exclusively for the *Rothia kristinae* YM-6 strain. These results confirm that even when a strain of the same species is present in a sample, only the *Rothia kristinae* YM-6 strain can be specifically and clearly detected and identified at strain level.

Another aspect provides a kit for detecting the *Rothia kristinae* YM-6 strain deposited under accession number KCCM12685P, including the composition according to one aspect.

The kit may further include reagents for a nucleic acid amplification reaction.

The nucleic acid amplification reaction may be any nucleic acid amplification technique known in the art, without limitation. The nucleic acid amplification reaction may be, for example, a polymerase chain reaction (PCR). The PCR may include real-time PCR, reverse transcriptase polymerase chain reaction (RT-PCR), quantitative real-time polymerase chain reaction (qRT-PCR), and the like, but is not limited thereto. Accordingly, the kit may be a PCR kit.

The reagents for the nucleic acid amplification reaction may include a DNA polymerase, dNTPs, a buffer solution, and the like.

The kit may further include a user guide describing optimal conditions for performing the reaction.

Another aspect provides a method of detecting the *Rothia kristinae* YM-6 strain deposited under accession number KCCM12685P using the composition, the primer set, or the kit according to one aspect.

The detection may be performed by at least one method selected from PCR analysis, DNA sequencing, hybridization by microarray, DNA amplification fingerprinting (DAF), Northern blot, and Southern blot, but is not limited thereto.

In an embodiment, the method may include isolating a nucleic acid from a sample;
performing a nucleic acid amplification reaction using the nucleic acid as a template and the primer set according to one aspect; and
detecting an amplification product generated by the nucleic acid amplification reaction.

The sample may be a strain to be analyzed or a substance suspected of containing the strain.

The nucleic acid may be DNA or RNA. The nucleic acid may be DNA. The DNA may include genomic DNA (gDNA). The DNA may include chromosomal DNA and plasmid DNA (pDNA). The genomic DNA may refer to chromosomal DNA.

A method known in the art may be used to isolate the nucleic acid from the sample.

Any nucleic acid amplification technique known in the art may be used without limitation as the nucleic acid amplification reaction. The nucleic acid amplification reaction may be, for example, PCR. The PCR may include real-time PCR, RT-PCR, qRT-PCR, and the like, but is not limited thereto.

The annealing temperature of the nucleic acid amplification reaction may be 63 to 70 °C, 63 to 69 °C, 64 to 70 °C, 64 to 69 °C, 65 to 70 °C, 65 to 69 °C, 66 to 70 °C, 66 to 69 °C, 67 to 70 °C, 67 to 69 °C, or about 68 °C.

Detection of the amplification product may be performed through a DNA chip, gel electrophoresis, radioactivity measurement, fluorescence measurement, phosphorescence measurement, magnetic field measurement, and the like, but is not limited thereto.

In the detection, when an amplification product having a length of 313 bp is detected, the sample may be identified as the *Rothia kristinae* YM-6 strain deposited under accession number KCCM12685P, or the strain may be identified as being present in the sample.

Accordingly, the method may be a method of identifying the *Rothia kristinae* YM-6 strain deposited under accession number KCCM12685P.

Overlapping contents are omitted in consideration of the complexity of the present specification, and terms not otherwise defined herein have meanings commonly used in the art to which the present disclosure pertains.

### Advantageous Effects of Invention

Since the composition according to one aspect utilizes a whole genome sequence (WGS)-based molecular marker, it can rapidly and accurately detect or identify the *Rothia kristinae* YM-6 strain with high detection specificity at strain or species level.

### Brief Description of Drawings

FIG. 1 shows results of confirming a selected primer pair for detecting *Rothia kristinae* YM-6 via NCBI Primer-BLAST.
FIG. 2 shows electrophoresis results confirming that a primer pair for detecting *Rothia kristinae* YM-6 can specifically detect only a target microorganism.

### Best Mode for Carrying out the Invention

### Mode for the Invention

Hereinafter, the present disclosure will be described in more detail through examples. However, these examples are provided to describe one or more embodiments by way of illustration, and the scope of the present disclosure is not limited to these examples.

### Example 1: Preparation of Primers

Primers capable of specifically detecting the *Rothia kristinae* YM-6 strain (accession number: KCCM12685P) were prepared.

First, whole genome sequences of microorganisms belonging to closely related strains or species that are otherwise indistinguishable by general physiological or chemical differences were collected from the National Center for Biotechnology Information (NCBI). The target strain and the comparative strains are shown in Table 1 below.

**[Table 1]**

| | **Genus** | **Species** | **Strain** |
|---|---|---|---|
| **Target strain** | *Rothia* | *kristinae* | YM-6 |
| **Comparative strain 1** | *Rothia* | *kristinae* | KACC 15198 |
| **Comparative strain 2** | *Kocuria* | *varians* | YM-3 |
| **Comparative strain 3** | *Kocuria* | *rhizophila* | KACC 14744 |
| **Comparative strain 4** | *Rothia* | *koreensis* | KACC 17049 |

After fragmentation was performed on the whole genome sequence information of the strains in Table 1 in units of 10 to 500 bp, numbers were assigned to the fragmented sequences using a Linux command. In addition, using NCBI Nucleotide BLAST, regions having identical sequences were excluded, and sequence information for the remaining regions was collected. Thereafter, candidate primers were designed using a primer design system such as NCBI Primer-BLAST from among the regions containing unique sequences.

### Example 2: In silico PCR of Candidate Primers

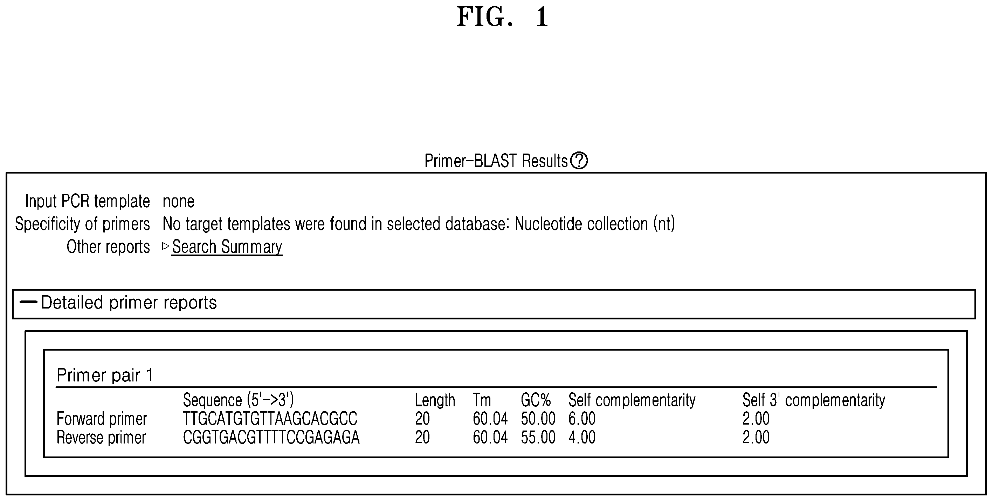

Prior to preparing the primers selected as candidates in Example 1, theoretical verification of the primers was performed via *in silico* PCR on a server. Results of confirming the finally selected primer pair via NCBI Primer-BLAST are shown in FIG. 1.

As a result, as shown in FIG. 1, the finally selected primer pair can specifically detect a sequence having a length of 313 bp at positions 5311 to 5623 of the complete sequence of the genomic DNA of the *Rothia kristinae* YM-6 strain. The sequence having a length of 313 bp is shown in SEQ ID NO: 1.

### Example 3: Performance of Primer Verification PCR at Optimal Temperature

For the primers finally selected in Example 2, a primer verification experiment was performed via PCR using the genomic DNA (gDNA) of the target strain and the comparative strains as templates at an optimal temperature. The sequences of the primer pair are shown in Table 2 below.

**[Table 2]**

| **Target strain** | **Direction** | **Sequence (5'->3')** | **SEQ ID NO.** | **Annealing temperature** |
|---|---|---|---|---|
| *Rothia kristinae* YM-6 | Forward | TTGCATGTGTTAAGCACGCC | 2 | 68 °C |
| | Reverse | CGGTGACGTTTTCCGAGAGA | 3 | |

### Example 4: Confirmation of Specific Reaction of Primers

An experiment was performed to confirm whether the finally selected primers bind specifically and exclusively to the target strain.

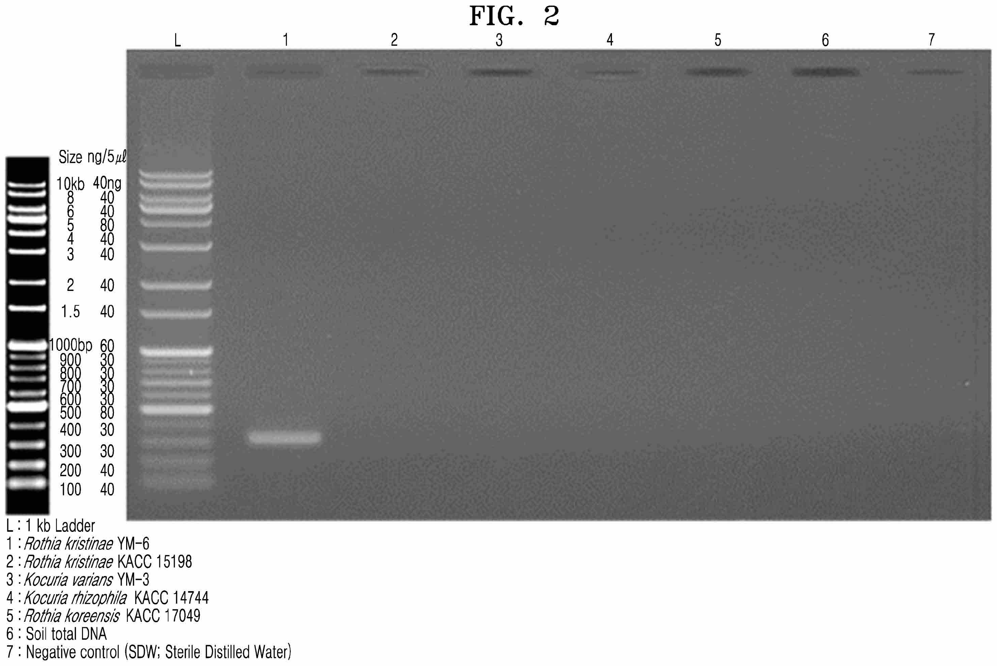

Specifically, PCR was performed using the corresponding primer pair on the respective gDNAs of *Rothia kristinae* YM-6, *Rothia kristinae* KACC 15198, *Kocuria varians* YM-3, *Kocuria rhizophila* KACC 14744, and *Rothia koreensis* KACC 17049; DNA of an environmental sample; and a negative control. Total DNA of soil was used as the DNA of the environmental sample. Sterile distilled water (SDW) was used as the negative control. Electrophoresis results of the PCR products are shown in FIG. 2.

As a result, as shown in FIG. 2, a DNA band of 313 bp was identified only in the PCR product using *Rothia kristinae* YM-6 as a sample.

Optimal PCR amplification conditions for detecting the *Rothia kristinae* YM-6 strain are shown in Table 3 below.

**[Table 3]**

| | **Temperature** | **Time** | **Cycles** |
|---|---|---|---|
| Pre-denaturation | 95 °C | 3 minutes | 1 cycle |
| Denaturation | 95 °C | 30 seconds | 30 cycles |
| Annealing | 68 °C | 30 seconds | |
| Extension | 72 °C | 30 seconds | |
| Final extension | 72 °C | 3 minutes | 1 cycle |
| Hold | 4 °C | - | - |

## Claims

1. A composition for detecting a *Rothia kristinae* YM-6 strain deposited under accession number KCCM12685P, comprising an agent capable of specifically detecting a polynucleotide consisting of SEQ ID NO: 1 or a complementary polynucleotide thereof.

2. The composition of claim 1, wherein the polynucleotide or the complementary polynucleotide thereof is a partial sequence of a whole genome sequence of the *Rothia kristinae* YM-6 strain.

3. The composition of claim 1, wherein the agent is a primer or probe that specifically binds to the polynucleotide or the complementary polynucleotide thereof.

4. The composition of claim 3, wherein the primer is a primer set consisting of a forward primer set forth in SEQ ID NO: 2 and a reverse primer set forth in SEQ ID NO: 3.

5. A primer set for detecting a *Rothia kristinae* YM-6 strain deposited under accession number KCCM12685P, consisting of a forward primer set forth in SEQ ID NO: 2 and a reverse primer set forth in SEQ ID NO: 3.

6. A kit for detecting a *Rothia kristinae* YM-6 strain deposited under accession number KCCM12685P, comprising the composition of claim 1.

7. The kit of claim 6, further comprising a reagent for a nucleic acid amplification reaction.

8. A method of detecting a *Rothia kristinae* YM-6 strain deposited under accession number KCCM12685P, comprising:
isolating a nucleic acid from a sample;
performing a nucleic acid amplification reaction using the nucleic acid as a template and the primer set of claim 5; and
detecting an amplification product generated by the nucleic acid amplification reaction

9. The method of claim 8, wherein the nucleic acid is DNA.

10. The method of claim 8, wherein when an amplification product having a length of 313 bp is detected, the sample is identified as the *Rothia kristinae* YM-6 strain deposited under accession number KCCM12685P, or the strain is identified as being present in the sample.
